# FASCICULE DE BREVET EUROPEEN

(11) **EP 1 473 041 B1**
(45) Date de publication et mention de la délivrance du brevet: **18.10.2006**
(21) Numéro de dépôt: 04007143.3
(22) Date de dépôt: 28.03.2002
(51) Int. Cl.: A61K 39/35, A61K 38/48, A61P 37/08

(54) **Composition pharmaceutique anti-allergique**
Anti-allergische pharmazeutische Zusammensetzung
Anti-allergic pharmaceutical composition

(30) Priorité: 30.03.2001 FR 0104370; 03.05.2001 FR 0105929; 29.05.2001 US 867159
(43) Date de publication de la demande: 03.11.2004
(62) Demande divisionnaire de: 02757745.1
(73) Titulaire: Antialis, 71230 Saint-Vallier (FR)
(72) Inventeur: Loria, Emile, 31500 Toulouse (FR); Terrasse, Gaetan, 71230 Saint-Villier (FR); Trehin, Yves, 31500 Toulouse (FR)
(74) Mandataire: Schmitt, John

(56) Documents cités:
- WO-A-00/50044
- WO-A-88/10297
- WO-A-94/27634
- GOUGH LUCY ET AL: "The cysteine protease activity of the major dust mite allergen Der p 1 selectively enhances the immunoglobulin E antibody response" JOURNAL OF EXPERIMENTAL MEDICINE, vol. 190, no. 12, 20 décembre 1999 (1999-12-20), pages 1897-1901, XP002298020 ISSN: 0022-1007
- ODA NARUHITO ET AL: "Long-term analysis of allergen-specific T cell clones from patients with asthma treated with allergen rush immunotherapy" CELLULAR IMMUNOLOGY, vol. 190, no. 1, 25 novembre 1998 (1998-11-25), pages 43-50, XP002298021 ISSN: 0008-8749
- JEANNIN P ET AL: "SPECIFIC HISTAMINE RELEASE CAPACITY OF PEPTIDES SELECTED FROM THE MODELIZED DER P I PROTEIN, A MAJOR ALLERGEN OF DERMATOPHAGOIDES PTERONYSSINUS" MOLECULAR IMMUNOLOGY, ELMSFORD, NY, US, vol. 29, no. 6, 1 juin 1992 (1992-06-01), pages 739-749, XP002025555 ISSN: 0161-5890

### SEQUENCE LISTING

<110> ANTIALIS
<120> Composition pharmaceutique anti-allergique
<130> B11812PCT-antialis
<140> PCT/FR02/xxxx
   <141> 2002-03-28
<150> FR01/04370
   <151> 2001-03-30
<150> FR01/05929
   <151> 2001-05-03
<150> US09/867,159
   <151> 2001-05-29
<160> 7
<170> PatentIn version 3.1
<210> 1
   <211> 666
   <212> DNA
   <213> Dermatophagoides pteronyssinus
   <400> 1
   actaacgcct gcagtatcaa tggaaatgct ccagctgaaa tcgatttgcg acaaatgcga 60
   actgtcactc ccattcgtat gcaaggaggc tgtggttcat gttgggcttt ctctggtgtt 120
   gccgcaactg aatcagctta tttggctcac cgtaatcaat cattggatct tgctgaacaa 180
   gaattagtcg attgtgcttc ccaacacggt tgtcatggtg ataccattcc acgtggtatt 240
   gaatacatcc aacataatgg tgtcgtccaa gaaagctact atcgatacgt tgcacgagaa 300
   caatcatgcc gaccaccaaa tgcacaacgt ttcggtatct caaactattg ccaaatttac 360
   ccaccaaatg caaacaaaat tcgtgaagct ttggctcaaa cccacagcgc tattgccgtc 420
   attattggca tcaaagattt agacgcattc cgtcattatg atggccgaac aatcattcaa 480
   cgcgataatg gttaccaacc aaactatcac gctgtcaaca ttgttggtta cagtaacgca 540
   caaggtgtcg attattggat cgtacgaaac agttgggata ccaattgggg tgataatggt 600
   tacggttatt ttgctgccaa catcgatttg atgatgattg aagaatatcc atatgttgtc 660
   attctc                                                            666
<210> 2
   <211> 222
   <212> PRT
   <213> Dermatophagoides pteronyssinus
<220>
   <221> PEPTIDE
   <222> (1)..(222)
   <223> Séquence peptidique cystine protéase.
<400 2
<210> 3
   <211> 10
   <212> PRT
   <213> Dermatophagoides pteronyssinus
<220>
   <221> PEPTIDE
   <222> (1)..(10)
   <223> Comporte l'épitope de cystine protéase.
<400> 3
   Arg Met Gln Gly Gly Cys Gly Ser Cys Asn
   1               5                   10
<210> 4
   <211> 10
   <212> PRT
   <213> Dermatophagoides pteronyssinus
<220>
   <221> peptide
   <222> (1)..(10)
   <223> Comporte épitope cystine protéase.
<400> 4
   Gln Pro Asn Tyr His Ala Val Asn Ile Val
   1               5                   10
<210> 5
   <211> 9
   <212> PRT
   <213> Dermatophagoides pteronyssinus
<220>
   <221> peptide
   <222> (1)..(9)
   <223> Comporte l'épitope cystine protéase.
<400> 5
   Trp Thr Val Arg Asn Ser Trp Asp Thr
   1 5
<210> 6
   <211> 9
   <212> DNA
   <213> Dermatophagoides pteronyssinus
<220>
   <221> amorce
   <222> (1)..(9)
   <223>
<400> 6
   gcggcggcg 9
<210> 7
   <211> 12
   <212> DNA
   <213> Dermatophagoides pteronyssinus
<220>
   <221> amorce
   <222> (1)..(12)
   <223>
<400> 7
   tgagcggcgg cg 12

## Description

La présente invention concerne de nouvelles compositions pharmaceutiques pour la prévention et le traitement de l'allergie. Les allergies constituent un fléau dont souffre 25% de la population mondiale. Ce nombre est en augmentation en liaison avec une évolution de la toxicité de l'environnement (poussière, aliments, véhicule automobile). En outre, le risque pour un individu de souffrir d'allergie augmente en cas d'antécédents allergiques chez les parents.

Le mécanisme biologique de l'allergie peut être décrit comme une réaction anormalement amplifiée suite à l'entrée de l'allergène dans l'organisme. Les évènements ci-après sont responsables de la réaction:
- identification de l'allergène par l'organisme,
- secrétion de cytokines en réponse à la pénétration des allergénes,
- stimulation des cellules Th0 privilégiant une différentiation accélérée des cellules Th0 en Th2 au lieu de leur différenciation normale équilibrée en Th1 et Th2
- les cellules Th2 synthétisent des Interleukines 4 et 13, responsables de l'aggravation des symptômes allergiques par la recrudescence de la synthèses des IgE ,
- la phase terminale de la réaction est la libération d'histamine et sérotonine avec un effet recruteur sur les clones Th2,
- réaction d'auto-entretien toxique et inflammatoire, même en dehors d'une stimulation antigénique.

Les cellules présentatrices d'antigène (CPA: macrophages, cellules dendritiques, lymphocytes B) participent à la réaction d'hypersensibilité par une coopération cellulaire fondamentale pour entraîner la réaction immune. L'allergie appartient à la classe des réactions de défense contre le non soi. Les principaux allergènes sont les acariens (80%) et les pollens (20%).

Les réactions d'auto-stimulation de clones CPA ont un effet sur le niveau général de libération d'histamine et sérotonine avec pour conséquence d'aggraver la symptomatologie générale clinique.

Le niveau de recrutement de nouvelles cellules sécrétant des IgE est ainsi accru facilitant alors l'explosion des signes cliniques lors de la pénétration d'un nouvel allergéne dans l'organisme. Ceci peut être observé chez l'atopique où les réactions allergiques sont violentes du fait d'un niveau élevé de clones Th 2 favorisant la synthése d' IgE.

La réaction générale observée du fait de la pénétration du nouvel allergéne n'est pas due à la toxicité de celui-ci, mais tout simplement au fait qu'il existe un niveau de déclenchement des phénomènes allergiques très bas favorisé par les autres sensibilisations.

Pour les allergologues, l'allergie est communément perçue comme une réaction due à une hypersynthése des immunoglobulines IgE. La réaction inflammatoire affecte principalement la sphère respiratoire et ORL, avec une focalisation pathologique au niveau du nez, des poumons et de la peau. Les pathologies associées à l'allergie sont invalidantes et souffrent du manque d'efficacité du traitement classique. Il n'y a pas de stratégie préventive et les moyens curatifs sont insuffisants ou mal utilisés.

Le traitement usuel de la maladie allergique consiste, dans un premier temps, en la reconnaissance de l'allergène responsable: acariens, pollen, moisissures, aliments ; dans un deuxième temps, on édicte des mesures d'éviction, de manière à éviter l'exposition du sujet à cet(ces) allergène(s) ; dans un troisième temps, un traitement est proposé dont le focus s'effectue sur l'organe cible qui apparaît symptomatique: traitement ORL sur une rhinite, traitement antiasthmatique si la sphère atteinte est la sphère respiratoire, traitement dermatologique si l'atteinte est cutanée.

En cas d'échec des mesures précédentes, on peut proposer des mesures thérapeutiques isolées ou complémentaires. Généralement, on prescrit au sujet des anti-histaminiques, mais ce traitement n'est pas suffisant pour faire disparaître tous les symptômes de l'allergie. Il est toutefois utile pour en réduire l'intensité, et la rendre plus supportable au patient. Egalement, une immunothérapie est généralement proposée. Cette immunothérapie se base sur l'identification de l'allergène, et son but est de soumettre le sujet progressivement à des taux de plus en plus élevés de l'allergène spécifique (pollen spécifique, acarien spécifique, moisissures spécifiques) considéré, de manière à « habituer » l'organisme à la présence de cet allergène. La complexité du traitement mis en route empêche une adhésion à ce dernier. La succession de thérapies est un facteur d'échec patent des traitements.

Les pathologies associées à l'allergie sont invalidantes et souffrent du manque d'efficacité des traitements classiques.

Les Inventeurs ont affiné la connaissance du mécanisme biologique de l'allergie en remarquant que la synthèse des immunoglobulines IgE en excès était, en amont, provoquée par l'augmentation du taux de cellules Th2 et le dérèglement de la différenciation des Th0 au profit des Th2. A l'encontre de la démarche habituelle de l'homme du métier, les inventeurs ont considéré l'allergie non comme une réaction spécifique à un antigène spécifique, mais comme un dérèglement immunitaire global dû à l'augmentation des Th2.

Le problème technique que les inventeurs ont alors identifié consistait à savoir comment éviter ce déséquilibre en faveur de la synthèse des cellules Th2 ; à savoir comment bloquer le déséquilibre de la différenciation préférée des cellules ThO en cellules Th2. La solution proposée par la présente invention a pour but d'offrir de nouveaux moyens de traitement des allergies à la fois préventif et curatif.

Ce but est atteint en traitant les deux versants principaux de la réaction immune:
- d'une part, l'amont de la réaction immune, qui après présentation de l'antigène aux CPA aboutit à une synthèse accrue des IgE responsables de l'auto recrutement des cellules de l'immunité et
- d'autre part, l'aval de la réaction immune, qui aboutit à la libération des médiateurs préformés, essentiellement l'histamine, responsables d'une part de la symptomatologie clinique finale et agissant d'autre part en stimulateur de la transformation des Th0 en Th2.

Le traitement selon l'invention présente l'avantage d'être non-spécifique de l'allergène qui est à l'origine de l'allergie et permet d'aborder d'une manière globale la maladie allergique sans se préoccuper de la spécificité de l'allergène. En effet, les compositions selon l'invention permettent de traiter un niveau de réactivité immune et non de proposer une immunothérapie spécifique. Ces compositions sont efficaces pour traiter les sujets pour qui l'allergie est clairement de nature héréditaire. L'objectif est de faire retrouver à l'organisme une homéostasie de défense silencieuse par rapport à son environnement.

L'invention concerne l'utilisation d'un premier allergène ou d'une molécule d'acide nucléique isolée comprenant au moins une séquence polynucléotidique codant pour ledit premier allergène, pour la préparation d'une composition pharmaceutique utile pour traiter ou prévenir une allergie provoquée par un second allergène différent du premier. Un premier allergène préféré est une cystine protéase d'un acarien. Suivant un mode de réalisation préféré de l'invention, le premier allergène est une cystine protéase d'un acarien et l'autre allergène n'est pas une cystine protéase, d'un acarien.

Suivant une variante que l'invention, l' allergène est choisi parmi les allergènes ou mélange d'allergènes majeurs d'acariens capables d'induire une réaction immune, notamment des acariens *D. Ptéronyssinus* et/ou *D. Farinae.* De préférence, cet allergène est une cystine protéase, ou au moins un épitope peptidique d'une cystine protéase. De préférérence, il s'agit d'un épitope peptidique d'une cystine protéase dont la séquence en acides aminés comprend la séquence SEQ ID NO : 2 dans la liste de séquences en annexe. Suivant un autre mode de réalisation de l'invention, l'allergène est un peptide ou un mélange de peptides choisi dans le groupe comprenant les peptides de séquences SEQ ID NO : 3, SEQ ID NO : 4, SEQ ID NO : 5 dans la liste de séquences en annexe.

L'allergène utilisé selon l'invention peut être naturel et obtenu par extraction des acariens *D. Ptéronyssinus* et/ou *D. Farinae.* Alternativement, il peut être synthétique et obtenu par synthèse peptidique suivant des techniques classiquement connues.

De préférence, les quantités d'allergène présentes dans la composition de l'invention sont de l'ordre de 1 à 1500 µg et de préférence de 10 à 150 µg.

Avantageusement, la molécule d'acide nucléique isolée comprenant au moins une séquence polynucléotidique codant pour ledit allergène, est un vecteur, notamment un vecteur adénoviral. Avantageusement, la séquence polynucléotidique codant pour l'allergène contenue dans la composition de l'invention, code au moins pour une cystine protéase de séquence SEQ ID NO : 2 dans la liste de séquences en annexe ou, suivant une variante de l'invention, au moins pour les trois épitopes peptidiques SEQ ID NO : 3, SEQ ID NO : 4, SEQ ID NO : 5 dans la liste de séquences en annexe. Avantageusement, cette séquence nucléotidique comprend une séquence correspondant à la séquence SEQ ID NO : 1 dans la liste de séquences en annexe, ou, suivant une variante de l'invention, au moins une des séquences SEQ ID No. : 6 ou SEQ ID No. : 7 dans la liste de séquences en annexe.

Suivant un mode de réalisation préféré, la composition selon l'invention est libérée sous forme de patch transcutané pour permettre un meilleur accès des allergènes utilisés et/ou des séquences polynucléotidiques codant pour lesdits allergènes, aux cellules présentatrices d'antigène.

Suivant une variante de réalisation, la composition selon l'invention est libérée sous forme mucosale, collyre, spray nasal ou bronchique.

Suivant une autre variante, la composition selon l'invention est libérée sous forme galénique à délitement programmé en mucosale ou sub-linguale et secondairement *per os.*

La composition pharmaceutique de l'invention est utile pour la préparation d'un médicament destiné au traitement ou à la prévention de la réaction d'hypersensibilité allergique.

La composition selon l'invention est également utile pour la préparation d'un médicament destiné au traitement ou a la prévention de l'asthme allergique, de la rhinite allergique, de l'eczéma atopique et allergique

La composition selon l'invention est encore utile pour la préparation d'un médicament destiné au traitement ou à la prévention des manifestations allergiques de l'enfant, du nourrisson et de l'adulte.

Les inventeurs ont basé leurs premières expérimentations sur la réaction terminale toxique et inflammatoire de l'allergie, qui s'auto-entretient en l'absence de stimulation antigènique. Cette réaction est habituellement traitée en bloquant la phase terminale de l'allergie, par administration d'un antihistaminique, qui agit de manière compétitive avec l'histamine pour la fixation sur les récepteurs à l'histamine. Le blocage des récepteurs à l'histamine est la voie effectrice principale. Ce blocage doit s'effectuer sur un temps suffisamment long pour qu'un retour (feed-back) négatif sur la synthèse de ces récepteurs s'effectue également. Les antihistaminiques sont les molécules anti-récepteurs de choix pour bloquer cette réaction terminale.

Comme indiqué ci-dessus, l'allergie s'accompagne également d'une synthèse accrue d'histamine qui est également une cause d'auto-entretien de la réaction inflammatoire terminale. Cette synthèse d'histamine peut éventuellement être contrôlée pour améliorer l'efficacité de la composition pharmaceutique proposée précédemment.

Les inventeurs ont ensuite constaté que l'administration d'un allergène, donnait des résultats d'une surprenante efficacité et ils ont mis au point les compositions selon l'invention qui permettent de disposer d'une nouvelle approche allergénique constituant une vaccination préventive et non spécifique du développement des maladies allergiques.

Avantageusement, cet allergène est choisi parmi les antigènes ou mélange d'antigènes majeurs d'acariens capables d'induire une réaction immune. En effet, les travaux réalisés dans le cadre de l'invention ont consisté à identifier puis à utiliser des antigènes ubiquitaires d'acariens.

Ces antigènes sont présents en quantité importante dans l'environnement et sont à l'origine du développement des réactions allergiques dans le monde. Deux acariens, *D. Ptéronyssinus* (DP) et *D. Farinae* (DF) sont les plus représentés dans l'environnement mondial.

L'invention envisage tout particulièrement comme allergène, une cy.stine protéase porteuse de l'antigénicité qui est identique à 90 % pour ces deux acariens. La séquence en acides aminés de la cystine protéase de *D*. *Ptéronyssinus* (DP) est représentée dans la liste de séquences en annexe respectivement sous le numéro SEQ ID NO : 2.

Les allergènes mis en oeuvre dans les compositions de l'invention peuvent être, soit des extraits obtenus à partir de matériel biologique brut, soit des protéines totalement ou partiellement purifiées, éventuellement produites par génie génétique, ou par synthèse peptidique.

Suivant un mode de réalisation particulier de l'invention, la composition comprend comme allergène, des épitopes peptidiques de la cystine protéase. Il a en effet été mis en évidence trois parties épitopiques qui sont identifiées comme étant des déclencheurs de la réaction immune. Il s'agit des trois peptides de séquences suivantes:

RMQGGCGSCN (SEQ ID NO : 3)

QPNYHAVNIV (SEQ ID NO : 4)

WTVRNSWDT (SEQ ID NO : 5)

et leurs possible analogues.

Les séquences des épitopes protéiques cités plus haut peuvent contenir des séquences supplémentaires en acides aminés ou des substitutions facilitant leur accrochage au Complexe Majeur d'Histocompatibilité (CMH).

L'invention envisagé donc tout spécialement des compositions pharmaceutiques comprenant comme allergène l'un au moins de ces trois peptides.

Ces épitopes peptidiques sont strictement identiques chez DF, DP, ainsi que chez d'autres acariens, car porteurs de la fonction enzymatique de la cystine protéase. Leur lipophilie, ainsi que le fait qu'elles supportent la fonction enzymatique, expliquent que ces parties épitopiques sont constantes d'une espèce d'acariens à une autre et les inventeurs considèrent qu'elle sont à l'origine d'une réaction immune générale.

L'utilisation de ces peptides, soit sous forme cyclisée, soit sous forme épigénique, voire sous la forme d'un acide nucléique (ADN ou ARN) comprenant une séquence nucléotidique codant pour l'un au moins de ces peptides, doit induire une tolérance à l'antigène naturel et diminuer le niveau général de la réaction immune amont.

La cyclisation des peptides et/ou leur inclusion dans une séquence plus importante permet une amélioration de la présentation des antigènes aux lymphocytes T. Cette amélioration de la présentation permettra une présentation des antigènes et des épitopes au CMH et par ce biais va déclencher la réaction immune de tolérance. En effet, les antigènes doivent au préalable être remaniés par les CPA. La forme épitopique simple ne permet pas un remaniement par les CPA car, d'une manière générale, seule une protéine plus longue que 10 acides aminées peut être découpée et présentée par les CPA aux lymphocytes T.

Ces peptides peuvent être associés à tout vecteur pharmaceutiquement acceptable par exemple de nature phospholipidique.

Lorsqu'il s'agit d'acides nucléiques, les séquences codant pour lesdits peptides peuvent être amplifiés par les séquences de nucléotides suivantes : 5'GCGGCGGCG 3' (SEQ ID NO : 6) ou 5' TGAGCGGCGGCG 3' (SEQ ID NO : 7).

Il est ainsi possible d'intégrer les épigènes correspondant aux épitopes de DP/DF avec une séquence d'amorces de nucléotides de séquence (SEQ ID NO : 7) en alternant ladite séquence (SEQ ID NO : 7) et un épitope de façon à intégrer les trois épitopes majeurs de DP/DF pris ensemble ou séparément.

L'intégration des épitopes pris ensemble conduit à avoir un ensemble constitué d'une séquence d'amorces de nucléotides (SEQ ID NO : 7) un premier épitope majeur, une séquence d'amorces de nucléotides (SEQ ID NO : 7), un deuxième épitope majeur, une séquence d'amorces de nucléotides (SEQ ID NO : 7), un troisième épitope majeur.

L'intégration des épitopes pris séparément conduit à mixer trois ensembles constitués chacun d'une séquence d'amorces de nucléotides (SEQ ID NO : 7) et d'un épitope majeur. Cette intégration des épitopes avec une séquence d'amorces de nucléotides selon la séquence suivante (SEQ ID NO : 7) doit améliorer l'efficacité de présentation des épigènes DP/DF aux lymphocytes T. Par cette présentation améliorée, les épigènes de DP/DF vont stimuler le switch TH1 et donc abaisser le niveau de réaction allergique.

L'utilisation de ces épitopes, ou d'une solution permettant le switch TH1/TH2 telle les amorces de nucléotides selon la séquence (SEQ ID NO : 7) constituent une solution efficace et innovante de prévention et traitement de l'allergie.

En conséquence, les compositions de l'invention comprennent une quantité efficace d'au moins un allergène comme défini précédemment sans présager du rôle de cet allergène dans la symptomatologie du patient.

Cette approche permet d'aborder d'une manière globale la maladie allergique sans se préoccuper de la spécificité de l'allergène. En effet, la composition selon l'invention permet de traiter un niveau de réactivité immune et non de proposer une immunothérapie spécifique.

La mise en oeuvre de l'allergène, sous les différentes formes décrites ci-dessus, dans les compositions selon l'invention, permet d'induire une tolérance à l'antigène naturel et diminue le niveau général de la réaction immune amont.

Les compositions selon l'invention comprennent une quantité d'allergènes de l'ordre de 1 à 1500 µg et avantageusement de 10 à 150 µg. Dans le cas des peptides, chacun de ceux-ci est avantageusement présent dans des proportions de l'ordre de 1 à 1500 µg de façon à freiner la réaction immunologique aboutissant à une synthèse accrue des IgE.

Les compositions selon l'invention peuvent se présenter sous une forme pour l'application transdermique, par exemple une pommade pour l'enfant, pour l'administration orale, par exemple lyoc à libération lente, ou encore des comprimés gastro-résistants ou des gommes. Il peut aussi s'agir de spray ou de collyre, ou de formes galéniques à délitement programmé en mucosal et secondairement *per os*.

Ainsi, les compositions de l'invention se prêtent à différents modes d'administration choisis en adaptation avec le profil pathologique du patient et son âge. Pour les enfants, la forme patch ou la forme sirop ou comprimé à sucer. Les autres formes collyre ou injection peuvent également être utilisées. Chez l'adulte, toutes les formes galéniques sont envisageables.

L'intérêt d'une forme couplée permet également une simplicité thérapeutique, une adhésion au traitement simplifiée et donc au final une meilleure réussite des traitements.

Cette solution permet également de prévenir la maladie allergique et pas seulement des états pathologiques patents. Les enfants de parents allergiques pourraient être la cible majeure de ces traitements de prévention. Il en résulte moins de durée d'hospitalisation, moins de traitement antibiotique et une qualité de vie améliorée.

En effet le rééquilibrage de la différenciation des cellules Th0 en cellules Th1 et Th2 doit intervenir le plus précocément possible pour être efficace, car chez le nourisson c'est la différenciation en faveur des cellules TH2 qui est prédominante, responsable d'une hyperéactivité à l'environnement. Le rééquilibrage TH2/TH1 doit être précoce pour être le plus durable possible car la stimulation antigénique par les antigénes de l'environnement (acariens,et bactéries) sont des stimulateurs de la voie TH2.

Ainsi, la composition pharmaceutique selon l'invention est particulièrement utile pour la préparation d'un médicament destiné au traitement de la réaction d'hypersensibilité allergique.

Avantageusement la composition pharmaceutique selon l'invention se présente sous une forme galénique à délitement programmé en mucosale ou sub linguale et secondairement *per os*.

La composition pharmaceutique selon l'invention est également utile pour la préparation d'un médicament destiné au traitement ou à la prévention de la réaction d'hypersensibilité allergique, au traitement ou a la prévention de l'asthme allergique de la rhinite allergique de l'eczéma atopique et allergique.

Enfin, la composition pharmaceutique selon l'invention est particulièrement utile pour la préparation d'un medicament destiné au traitement ou à la prévention des manifestations allergiques de l'enfant, du nourrisson et de l'adulte.

D'autres avantages et caractéristiques de l'invention apparaîtront à la lecture des observations cliniques concernant le traitement de patients allergiques selon le tableau cité plus loin.

Ces observations ont été effectuées sur une centaine de patients, auxquels on a administré une composition selon l'invention associant au moins un composé anti-histaminique, un inhibiteur de synthèse d'histamine et un allergène.

Les patients ont un âge compris entre 7 et 60 ans. Ils présentent au moins un test positif acarien ou pollen, testé par prick test, ainsi qu'une symptomatologie de rhinite ou d'asthme depuis au moins un an.

Le profil pathologique des patients est classé selon la typologie suivante comprenant trois catégories descriptives: l'inflammation, la sécretion et l'élément figuré.
- Seul l'examen clinique permet de classer l'inflammation. On considère qu'il y a inflammation quand l'examen des muqueuses ou des organes cibles présente une coloration rouge signant ainsi un phénomène inflammatoire.
- La sécrétion concerne l'observation d'un exudat purulent ou non qui atteint un organe cible (muqueuses, peau, etc.).
- L'élément figuré est une modification de la structure de l'organe considéré qui peut se présenter sous plusieurs formes pathologiques. On ne tient compte que de la présence de cette modification sans entrer dans le détail de cette modification.

Le classement de la gravité pathologique se fait sur 4 niveaux distinguant l'intensité de l'atteinte selon une classification allant de 1 à 4, sous forme de fraction 1/4, 1/2 ou de nombre entier.

Ainsi, selon ce classement, une notation de 1/4 signifie une atteinte de l'organe cible compris entre 0 et inférieure à 1/4. Pour une notation de 1/2, ce classement signifie que l'atteinte est comprise entre 1/4 et la moitié de l'organe cible; pour 3/4, cette notation signifie que l'atteinte est supérieure à 1/2 et inférieure à 3/4; pour 1, elle signifie que l'atteinte est supérieure à 3/4.

Une première catégorie d'organes cibles est classée selon cette typologie. Elle comprend les yeux, le nez, le pharynx, le larynx et la peau.

Pour le poumon, la classification se fait selon les résultats d'une exploration fonctionnelle respiratoire avec des chiffres exprimés en pourcentage par rapport à la valeur normale (selon une classification internationale tenant compte en particulier de l'âge et de la taille).

Les patients sont suivis avec visite au moins à 2 mois, à 8 mois, à 12 mois ,à 24 mois. L'évolution des thérapeutiques prises et le nombre d'unités prises sont analysés.

Le tableau I ci-dessous donne une claire indication des résultats très positifs obtenus après environ 8 mois de traitement. On constate une nette amélioration de l'état pathologique des patients avec un score clinique total de gravité passant en moyenne d'un indice 9,56 à un indice 2,47 et un écart-moyen évoluant de 1,15 à 0,53 confirmant l'efficacité du traitement sur toutes les classes d'âge et de sexe des patients. La moyenne du nombre d'organes cibles atteints passe de 3,69 à 1,73 tandis que l'écart moyen des nombres d'organes cible atteints est réduit de 0,49 à 0,41.

**Tableau I**

| | | | | | Consultation initiale | | 3ème consultation après 8 mois de traitement | |
|---|---|---|---|---|---|---|---|---|
| Référence Patient | Sexe | Date de Naissance | DATE DE CONSULTATION INITIALE | Nb de TESTS + | Nb organes Cibles atteints | Score clinique total | Nb organes Cibles atteints | Score clinique total |
| 1 | M | 1964 | 1996 | 3 | 3 | 7 | 2 | 2 |
| 2 | F | 1936 | 2000 | 4 | 3 | 6 | 1 | 2 |
| 3 | F | 1944 | 1993 | 8 | 4 | 10 | 2 | 2 |
| 4 | F | 1974 | 1997 | 8 | 4 | 9 | 1 | 3 |
| 5 | F | 1950 | 1997 | 8 | 4 | 9 | 2 | 3 |
| 6 | M | 1960 | 1997 | 7 | 4 | 8 | 1 | 2 |
| 7 | F | 1944 | 1996 | 4 | 3 | 6 | 2 | 2 |
| 8 | F | 1963 | 1993 | 4 | 5 | 10 | 1 | 2 |
| 9 | M | 1988 | 1993 | 7 | 4 | 8 | 2 | 2 |
| 10 | M | 1991 | 1993 | 3 | 4 | 9 | 1 | 2 |
| 11 | M | 1971 | 2000 | 6 | 3 | 9 | 1 | 2 |
| 12 | M | 1948 | 2000 | 3 | 4 | 9 | 1 | 2 |
| 13 | M | 1929 | 2000 | 3 | 3 | 7 | 2 | 2 |
| 14 | M | 1953 | 1999 | 5 | 4 | 9 | 1 | 1 |
| 15 | F | 1932 | 1994 | 10 | 4 | 10 | 1 | 2 |
| 16 | F | 1934 | 1996 | 8 | 6 | 11 | 2 | 2 |
| 17 | F | 1982 | 1993 | 5 | 4 | 10 | 2 | 2 |
| 18 | F | 1968 | 1994 | 4 | 4 | 10 | 2 | 2 |
| 19 | M | 1996 | 1996 | 4 | 4 | 10 | 1 | 3 |
| 20 | F | 1991 | 1997 | 5 | 4 | 10 | 2 | 3 |
| 21 | F | 1990 | 1996 | 7 | 3 | 8 | 1 | 2 |
| 22 | F | 1949 | 2000 | 4 | 4 | 8 | 2 | 3 |
| 23 | M | 1995 | 2000 | 3 | 2 | 6. | 1 | 2 |
| 24 | F | 1961 | 1994 | 8 | 3 | 8 | 1 | 2 |
| 25 | M | 1987 | 1994 | 7 | 4 | 9 | 2 | 3 |
| 26 | F | 1991 | 1995 | 8 | 3 | 8 | 1 | 2 |
| 27 | M | 1967 | 1994 | 7 | 3 | 9 | 2 | 2 |
| 28 | M | 1989 | 1994 | 7 | 4 | 9 | 2 | 3 |
| 29 | M | 1947 | 1999 | 5 | 4 | 9 | 2 | 2 |
| 30 | F | 1920 | 1999 | 2 | 3 | 8 | 1 | 2 |
| 31 | F | 1963 | 1997 | 6 | 4 | 9 | 2 | 2 |
| 32 | M | 1979 | 1998 | 4 | 4 | 9 | 1 | 2 |
| 33 | F | 1983 | 2000 | 3 | 3 | 8 | 2 | 2 |
| 34 | M | 1996 | 1999 | 7 | 4 | 8 | 2 | 2 |
| 35 | F | 1946 | 1995 | 7 | 3 | 8 | 2 | 3 |
| 36 | F | 1958 | 1995 | 5 | 4 | 10 | 2 | 2 |
| 37 | F | 1946 | 1997 | 6 | 4 | 11 | 2 | 2 |
| 38 | F | 1965 | 1993 | 3 | 3 | 9 | 1 | 2 |
| 39 | M | 1973 | 2000 | 7 | 4 | 9 | 2 | 2 |
| 40 | M | 1957 | 1995 | 5 | 4 | 9 | 2 | 2 |
| 41 | F | 1942 | 1995 | 8 | 4 | 9 | 2 | 2 |
| 42 | F | 1933 | 1999 | 4 | 3 | 9 | 1 | 3 |
| 43 | F | 1959 | 1999 | 4 | 3 | 8 | 2 | 3 |
| 44 | F | 1965 | 1999 | 3 | 4 | 10 | 2 | 2 |
| 45 | F | 1944 | 1999 | 3 | 4 | 10 | 2 | 3 |
| 96 | F | 1942 | 1996 | 6 | 4 | 11 | 1 | 3 |
| 47 | F | 1948 | 1997 | 6 | 4 | 11 | 2 | 3 |
| 48 | F | 1963 | 1999 | 4 | 4 | 10 | 2 | 2 |
| 49 | M | 1981 | 1999 | 5 | 4 | 12 | 2 | 2 |
| 50 | M | 1995 | 2000 | 5 | 4 | 12 | 2 | 2 |
| 51 | M | 1989 | 1999 | 5 | 4 | 10 | 2 | 2 |
| 52 | M | 1997 | 1998 | 4 | 4 | 10 | 2 | 3 |
| 53 | F | 1997 | 1998 | 5 | 4 | 9 | 1 | 3 |
| 54 | F | 1995 | 1997 | 4 | 4 | 10 | 2 | 3 |
| 55 | F | 1984 | 1993 | 3 | 3 | 9 | 1 | 2 |
| 56 | M | 1969 | 1996 | 10 | 4 | 12 | 2 | 3 |
| 57 | M | 1951 | 1996 | 11 | 4 | 11 | 2 | 2 |
| 58 | M | 1992 | 1997 | 5 | 4 | 11 | 2 | 3 |
| 59 | M | 1975 | 1994 | 4 | 3 | 9 | 1 | 2 |
| 60 | M | 1977 | 2000 | 5 | 4 | 12 | 2 | 3 |
| 61 | M | 1989 | 1993 | 5 | 4 | 12 | 2 | 3 |
| 62 | M | 1994 | 1998 | 8 | 4 | 11 | 2 | 3 |
| 63 | F | 1993 | 1998 | 7 | 4 | 10 | 2 | 2 |
| 64 | F | 1988 | 1993 | 3 | 3 | 9 | 2 | 3 |
| 65 | F | 1940 | 1999 | 4 | 4 | 11 | 2 | 2 |
| 72 | F | 1951 | 2000 | 6 | 4 | 11 | 2 | 3 |
| 73 | F | 1956 | 1999 | 5 | 4 | 11 | 2 | 3 |
| 74 | M | 1982 | 1994 | 4 | 3 | 9 | 2 | 3 |
| 75 | F | 1944 | 1998 | 3 | 4 | 12 | 2 | 2 |
| 76 | F | 1992 | 1997 | 7 | 3 | 9 | 2 | 3 |
| 77 | M | 1997 | 1993 | 4 | 3 | 9 | 1 | 3 |
| 78 | F | 1955 | 1997 | 5 | 4 | 10 | 2 | 3 |
| 79 | F | 1996 | 1999 | 4 | 3 | 8 | 2 | 3 |
| 80 | F | 1936 | 1993 | 5 | 4 | 10 | 1 | 2 |
| 81 | M | 1949 | 1998 | 5 | 3 | 10 | 2 | 2 |
| 82 | M | 1966 | 1993 | 4 | 3 | 9 | 2 | 2 |
| 83 | F | 1963 | 2000 | 5 | 4 | 10 | 1 | 2 |
| 84 | F | 1954 | 1993 | 5 | 4 | 11 | 2 | 2 |
| 85 | F | 1995 | 2000 | 4 | 3 | 9 | 2 | 3 |
| 86 | M | 1988 | 1994 | 6 | 3 | 8 | 2 | 2 |
| 87 | F | 1969 | 1997 | 6 | 4 | 9 | 2 | 3. |
| 88 | M | 1963 | 1993 | 5 | 4 | 9 | 2 | 2 |
| 89 | M | 1994 | 1998 | 7 | 4 | 10 | 1 | 3 |
| 90 | F | 1992 | 1997 | 6 | 3 | 9 | 3 | 3 |
| 91 | M | 1988 | 1999 | 6 | 4 | 11 | 2 | 3 |
| 92 | M | 1955 | 1993 | 6 | 4 | 11 | 2 | 3 |
| 93 | M | 1944 | 1996 | 7 | 4 | 13 | 2 | 3 |
| 94 | M | 1986 | 1994 | 6 | 4 | 12 | 2 | 3 |
| 95 | M | 1954 | 1996 | 6 | 4 | 11 | 2 | 3 |
| 96 | F | 1989 | 1993 | 6 | 4 | 12 | 2 | 2 |
| 97 | M | 1965 | 1995 | 6 | 3 | 8 | 2 | 3 |
| 98 | M | 1986 | 1994 | 4 | 3 | 9 | 2 | 4 |
| 99 | F | 1956 | 1995 | 4 | 4 | 10 | 2 | 3 |
| 100 | F | 1944 | 1993 | 2 | 3 | 9 | 1 | 3 |
| 101 | F | 1995 | 1998 | 5 | 3 | 9 | 2 | 4 |
| 102 | M | 1960 | 1996 | 3 | 3 | 8 | 2 | 3 |
| 103 | F | 1928 | 1995 | 6 | 4 | 10 | 2 | 3 |

Le Tableau II ci-dessous montre la moyenne des scores cliniques ainsi que l'écart moyen des scores obtenus.

**Tableau II**

| | VISITE INITIALE | VISITE A 8 MOIS |
|---|---|---|
| MOYENNE DES SCORES CLINIQUES | 9,56 | 2,47 |
| ECART-MOYEN DES SCORES | 1,15 | 0,53 |

Le Tableau III ci-après illustre la moyenne du nombre d'organes cibles atteints ainsi que l'écart moyen du nombre d'organes cibles atteints.

**Tableau III**

| | VISITE INITIALE | VISITE A 8 MOIS |
|---|---|---|
| MOYENNE DU NB D'ORGANES CIBLE ATTEINTS | 3,69 | 1,73 |
| ECART-MOYEN DU NB D'O.C. ATTEINTS | 0,49 | 0,41 |

## Revendications

1. Utilisation d'un premier allergène ou d'une molécule d'acide nucléique isolée comprenant au moins une séquence polynucléotidique codant pour ledit premier allergène, pour la préparation d'une composition pharmaceutique déstinée au traitement ou à la prévention d'une allergie provoquée par un second allergène différent du premier

2. Utilisation selon la revendication 1 **caractérisée en ce que** ledit premier allergène est une cystine protéase d'un acarien.

3. Utilisation selon la revendication 1 **caractérisée en ce que** ledit premier allergène est au moins un épitope peptidique d'une cystine protéase.

4. Utilisation selon la revendication 3 **caractérisée en ce que** ledit premier allergène est au moins un épitope peptidique d'une cystine protéase de séquence SQ ID NO : 2 dans la liste de séquence en annexe.

5. Utilisation selon la revendication 3 **caractérisée en ce que** ledit premier allergène est un peptide ou un mélange de peptides choisi dans le groupe comprenant les peptides de séquence SQ ID NO : 3, SQ ID NO : 4, SQ ID NO : 5 dans la liste de séquence en annexe.

6. Utilisation selon la revendication 1 **caractérisée en ce que** ledit premier allergène est naturel et obtenu par extraction des acariens *D. Ptéronyssinus* et/ou *D. Férinae.*

7. Utilisation selon la revendication 2 **caractérisée en ce que** ledit premier allergène est une cystine protéase d'un acarien, et **en ce que** ledit autre allergène n'est pas une cystine protéase dudit acarien.

## Claims

1. Use of a first allergen or of an isolated nucleic acid molecule comprising at least one polynucleotide sequence encoding said first allergen, for preparing a pharmaceutical composition for use in the treatment or the prevention of an allergy caused by a second allergen different from the first.

2. Use according to Claim 1, **characterized in that** said first allergen is a cystine protease of an acarid.

3. Use according to Claim 1, **characterized in that** said first allergen is at least one peptide epitope of a cystine protease.

4. Use according to Claim 3, **characterized in that** said first allergen is at least one peptide epitope of a cystine protease of sequence SEQ ID NO: 2 in the sequence listing in the appendix.

5. Use according to Claim 3, **characterized in that** said first allergen is a peptide or a mixture of peptides, chosen from the group comprising the peptides of sequence SEQ ID NO: 3, SEQ ID NO: 4, SEQ ID NO: 5 in the sequence listing in the appendix.

6. Use according to Claim 1, **characterized in that** said first allergen is natural and is obtained by extraction of the acarids *D. pteronyssinus* and/or *D. farinae.*

7. Use according to Claim 2, **characterized in that** said first allergen is a cystine protease of an acarid, and **in that** said other allergen is not a cystine protease of said acarid.

## Patentansprüche

1. Verwendung eines ersten Allergens oder eines isolierten Nukleinsäuremoleküls, das mindestens eine Polynukleotidsequenz umfasst, die für das erste Allergen kodiert, zur Herstellung einer pharmazeutischen Zusammensetzung, die zur Behandlung oder zur Vorbeugung einer Allergie bestimmt ist, die durch ein zweites Allergen hervorgerufen wird, das sich vom ersten unterscheidet.

2. Verwendung nach Anspruch 1, **dadurch gekennzeichnet, dass** das erste Allergen eine Cystinprotease einer Milbe ist.

3. Verwendung nach Anspruch 1, **dadurch gekennzeichnet, dass** das erste Allergen mindestens ein Peptidepitop einer Cystinprotease ist.

4. Verwendung nach Anspruch 3, **dadurch gekennzeichnet, dass** das erste Allergen mindestens ein Peptidepitop einer Cystinprotease der Sequenz SQ-ID-NO: 2 aus dem Sequenzprotokoll im Anhang ist.

5. Verwendung nach Anspruch 3, **dadurch gekennzeichnet, dass** das erste Allergen ein Peptid oder ein Gemisch von Peptiden ist, ausgewählt aus der Gruppe, die die Peptide der Sequenz SQ-ID-NO: 3, SQ-ID-NO: 4, SQ-ID-NO: 5 aus dem Sequenzprotokoll im Anhang umfasst.

6. Verwendung nach Anspruch 1, **dadurch gekennzeichnet, dass** das erste Allergen natürlich ist und durch Extraktion aus den Milben *D. Pteronyssinus* und/oder *D. Farinae* erhalten wird.

7. Verwendung nach Anspruch 2, **dadurch gekennzeichnet, dass** das erste Allergen eine Cystinprotease einer Milbe ist, und dadurch, dass das andere Allergen keine Cystinprotease dieser Milbe ist.
